# FASCICULE DE BREVET EUROPEEN

(11) **EP 1 934 456 B1**
(45) Date de publication et mention de la délivrance du brevet: **01.04.2009**
(21) Numéro de dépôt: 06794374.6
(22) Date de dépôt: 01.09.2006
(51) Int. Cl.: F02K 1/38, F02K 1/48, F02K 3/06

(54) **Turbomoteur à double flux muni d'un dispositif pour atténuer le bruit de jet**
Mantelstromtriebwerk mit Vorrichtung zur Unterdrückung des Strahllärms
Turbofan engine with a device for attenuating the jet noise

(30) Priorité: 12.09.2005 FR 0509260
(43) Date de publication de la demande: 25.06.2008
(73) Titulaire: AIRBUS France, 31060 Toulouse Cédex (FR)
(72) Inventeur: PORTE, Alain, F-31770 Colomiers (FR)
(74) Mandataire: Bonnetat, Christian
(86) Numéro de dépôt international: PCT/FR2006/002016
(87) Numéro de publication internationale: WO 2007/031618

(56) Documents cités:
- WO-A-02/29232
- GB-A- 766 985
- GB-A- 768 553
- GB-A- 2 289 921
- GB-A- 2 372 779

## Description

La présente invention concerne un turbomoteur à double flux à bruit atténué.

On sait-que, à l'arrière d'un turbomoteur à double flux, le flux froid et le flux chaud s'écoulent dans le même sens vers l'aval dudit turbomoteur et entrent en contact, non seulement l'un avec l'autre, mais encore avec l'air ambiant. Comme les vitesses desdits flux sont différentes l'une de l'autre et de celle de l'air ambiant, il en résulte des cisaillements fluides de pénétration entre lesdits flux et entre ceux-ci et l'air ambiant, lesdits cisaillements fluides engendrant du bruit, appelé "bruit de jet" dans la technique aéronautique.

Pour atténuer un tel bruit de jet, on a déjà pensé à engendrer des turbulences aux frontières entre les fluides ayant des vitesses différentes. C'est ainsi que l'on a déjà proposé de pratiquer des échancrures dans le bord de sortie du flux chaud et/ou dans le bord de sortie du flux froid (voir par exemple GB-2 289 921). De telles échancrures sont réparties à la périphérie dudit bord de sortie et chacune d'elles présente généralement la forme au moins approximative d'un triangle dont la base est confondue avec le bord de sortie correspondant et dont le sommet se trouve en avant de ce bord de-sortie. Ces échancrures sont généralement appelées "chevrons" dans la technique aéronautique. Bien entendu, lesdits "chevrons" sont deux à deux-séparés par une "protubérance".

Ces échancrures et protubérances connues sont efficaces pour atténuer le bruit de jet ; cependant, elles présentent l'inconvénient d'engendrer une traînée importante.

Par ailleurs, le document GB-2 372 779 constate qu'une atténuation du bruit de jet n'est pas nécessaire en vol de croisière et que, en conséquence, il est avantageux, notamment en ce qui concerne la traînée, de rendre lesdites protubérances mobiles de façon qu'elles puissent prendre :
- soit une position déployée saillante, utilisée au décollage et à l'atterrissage, pour laquelle elles sont aptes à atténuer le bruit de jet ;
- soit une position rétractée, utilisée en croisière, pour laquelle elles n'exercent aucune action d'atténuation du bruit de jet.

Pour ce faire, dans GB-2 372 779, ladite surface annulaire externe ou ladite surface annulaire interne de ladite partie arrière du capot entourant le générateur de flux chaud est rendue partiellement mobile sous l'action d'un mécanisme d'actionnement spécialement prévu à cet effet. On remarquera que, en position déployée desdites protubérances, les échancrures sont traversantes (non obturées par la surface annulaire qui ne porte pas les échancrures) et que, en position rétractée desdites protubérances, lesdites échancrures sont borgnes (obturées par celle desdites surfaces annulaires qui ne porte pas les échancrures).

Ainsi, la leçon technique du document GB-2 372 779 comporte les deux propositions suivantes :
a/ l'atténuation du bruit de jet n'est obtenue qu'avec des échancrures traversantes ; et
b/ aucune atténuation du bruit de jet n'est possible si les échancrures sont borgnes.

Aussi, l'objet de la présente invention est de remédier aux inconvénients de la technique antérieure rappelée ci-dessus en prévoyant des échancrures, qui sont aussi efficaces que les chevrons connus en ce qui concerne l'atténuation du bruit de jet, mais qui engendrent une traînée bien inférieure, et permettant d'obtenir l'atténuation de bruit de jet recherchée sans les complications ni les augmentations de masse résultant :
- d'une mobilité partielle nécessaire d'une surface annulaire de la partie arrière du capot entourant le générateur de flux chaud ; et
- d'un mécanisme spécial pour l'actionnement de la partie mobile desdites surfaces annulaires.

L'objet de l'invention est défini dans la revendication 1, qui est remarquable en ce que lesdites échancrures sont borgnes, du fait qu'elles entament l'une desdites surfaces annulaires convergentes formant ledit orifice de sortie sans entamer l'autre desdites surfaces annulaires convergentes.

En effet, de façon étonnante et contrairement à l'enseignement du document GB-2 372 779, la demanderesse a trouvé que des échancrures borgnes fixes étaient aptes à permettre l'obtention d'une atténuation de bruit de jet désirée, avec réduction de traînée.

Selon d'autres particularités de l'invention, en fonction des caractéristiques du turbomoteur considéré :
- les échancrures borgnes peuvent entamer ladite surface annulaire externe sans entamer ladite surface annulaire interne ou bien, au contraire, elles peuvent entamer ladite surface annulaire interne sans entamer ladite surface annulaire externe ; et
- seul l'orifice de sortie du flux chaud est pourvu desdites échancrures borgnes ou bien, au contraire, seul l'orifice de sortie du flux froid est pourvu desdites échancrures borgnes ; à moins que l'orifice de sortie du flux chaud et l'orifice de sortie du flux froid soient tous deux pourvus de telles échancrures borgnes.

Dans le cas usuel où ledit système de capot est parcouru par de l'air de ventilation destiné à réguler la température dudit générateur de flux chaud, cet air de ventilation peut s'échapper en totalité à travers lesdites échancrures borgnes prévues dans le bord de l'orifice de sortie du flux chaud. Cependant, ce bord peut, de façon connue, comporter une fente périphérique d'échappement, l'air de ventilation ne s'échappant alors que partiellement à travers lesdites échancrures adjacentes à ladite fente.

Par ailleurs, de façon connue, l'orifice de sortie concerné peut être délimité par un arêtier annulaire, duquel sont solidaires lesdites surfaces annulaires externe et interne convergentes. Il est alors avantageux que lesdites échancrures borgnes soient formées par des évidements pratiqués dans l'une des faces dudit arêtier.

Les figures du dessin annexé feront bien comprendre comment l'invention peut être réalisée. Sur ces figures, des références identiques désignent des éléments semblables.

La figure 1 représente, en coupe axiale schématique, un turbomoteur connu, destiné à être perfectionné par la mise en oeuvre du procédé de la présente invention.

La figure 2 illustre, en perspective schématique, un premier mode de mise en oeuvre pour le bord de sortie d'un flux d'un turbomoteur à double flux perfectionné selon l'invention.

La figure 3 est une coupe schématique selon la ligne III-III de la figure 2.

La figure 4 illustre, en perspective schématique, un deuxième mode de mise en oeuvre-pour le bord de sortie d'un flux d'un turbomoteur à double flux perfectionné selon l'invention.

La figure 5 est une coupe schématique selon la ligne V-V de la figure 4.

La figure 6 illustre, en perspective schématique, un troisième mode de mise en oeuvre pour le bord de sortie d'un flux d'un turbomoteur à double flux perfectionné selon l'invention.

La figure 7 est une coupe schématique selon la ligne VII-VII de la figure 6.

La figure 8 illustre, en perspective schématique, un quatrième mode de réalisation pour le bord de sortie d'un flux d'un turbomoteur à double flux perfectionné selon l'invention.

La figure 9 est une coupe schématique selon la ligne IX-IX de la figure 8.

La figure 10 montre schématiquement en coupe un mode de réalisation pratique pour les variantes des figures 2, 3, 6 et 7.

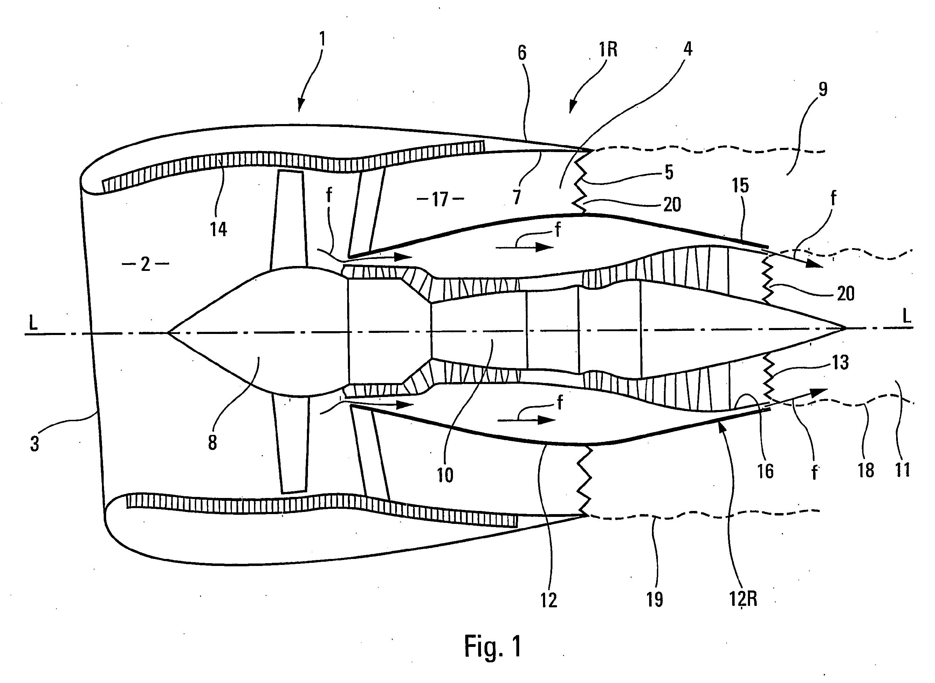

Le turbomoteur à double flux de type connu, montré par la figure 1, comporte une nacelle creuse 1, d'axe longitudinal L-L, comportant, à l'avant, une entrée d'air 2 pourvue d'un bord d'attaque 3 et, dans sa partie arrière 1R, une sortie d'air annulaire 4 pourvue d'un bord de fuite 5. La partie arrière 1R comprend une surface annulaire externe 6 et une surface annulaire interne 7 convergeant l'une vers l'autre pour former ledit bord de fuite 5.

A l'intérieur de ladite nacelle creuse 1, sont disposés :
- une soufflante 8 dirigée vers l'entrée d'air 2 et apte à engendrer le flux froid 9 pour le turbomoteur ;
- un générateur central 10, comprenant de façon connue des compresseurs à basse et haute pression, une chambre de combustion et des turbines à basse et haute pression, et engendrant le flux chaud 11 dudit turbomoteur :
- un système de capot 12 entourant ledit générateur de flux chaud 10 et pourvu, dans sa partie arrière 12R, d'un bord de sortie 13 pour le flux chaud 11 ; et
- des revêtements d'atténuation acoustique 14, destinés à absorber les bruits internes engendrés par la soufflante 8 et le générateur de flux chaud 10.

La partie arrière 12R du système de capot 12 comprend une surface annulaire externe 15 et une surface annulaire interne 16 convergeant l'une vers l'autre pour former ledit bord de sortie 13 du flux chaud 11. De plus, ledit système de capot 12 délimite avec la nacelle 1 un canal interne 17 à-section annulaire, aboutissant à la sortie d'air 4. Le flux froid traverse le canal interne 17 et la sortie 4 et sort à travers le bord de fuite 5, lui servant de bord de sortie.

Ainsi, à la sortie de ce turbomoteur connu, le flux chaud central 11 est entouré par le flux froid annulaire 9, qui pénètre dans l'air ambiant. Sur la figure 1, on a représenté schématiquement la frontière 18 entre le flux chaud et le flux froid 9 et la frontière 19 entre le flux froid 9 et l'air ambiant. Bien entendu, aux frontières 18 et 19, les fluides en contact ont des vitesses différentes, ce qui engendre le bruit de jet décrit ci-dessus.

Pour atténuer ce bruit de jet, le bord de sortie 13 du flux chaud et/ou lé bord de sortie 5 du flux froid sont pourvus, de façon connue, d'échancrures 20 réparties à leur périphérie. Ces échancrures 20 traversent toute l'épaisseur -desdits bords de sortie 13, 5 et engendrent une traînée importante.

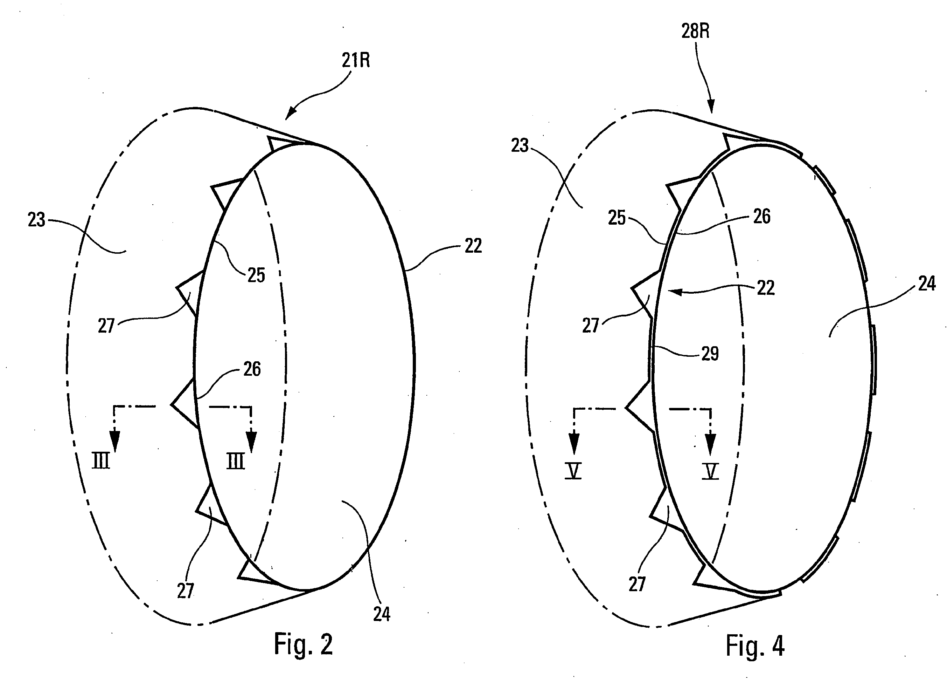

Afin de remédier à ce dernier inconvénient, la présente invention supprime les échancrures traversantes 20, comme cela est représenté sur les figures 2 à 10.

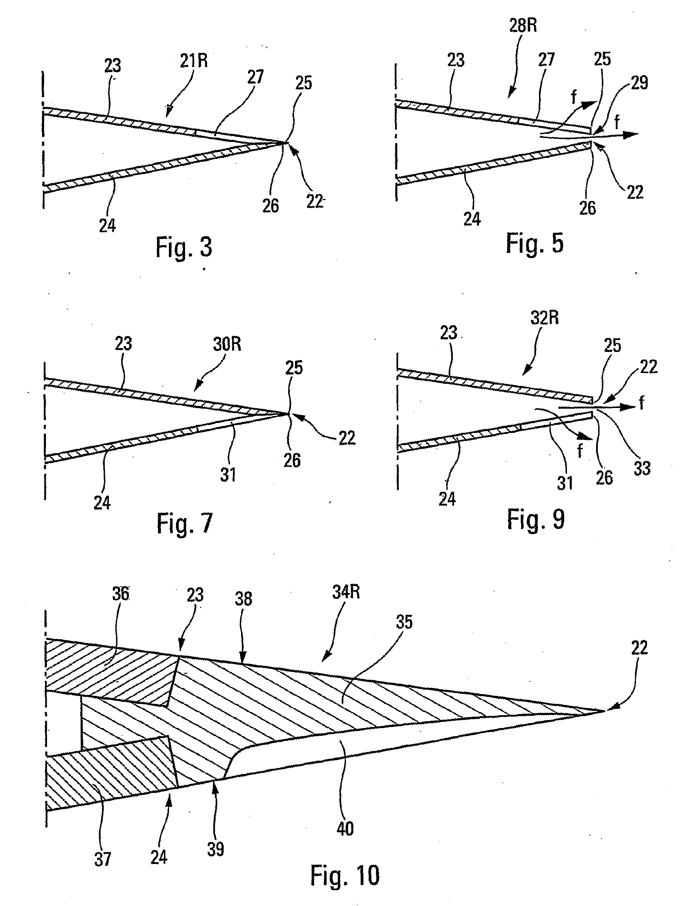

Sur les figures 2 et 3, on a représenté un premier mode de réalisation de partie arrière 21R à orifice de sortie 22, apte à perfectionner, conformément à l'invention, l'une ou l'autre des parties arrière 1R et 12R.

La partie arrière 21R des figures 2 et 3 comporte une surface annulaire externe 23 (comparable aux surfaces annulaires externes 6 et 15) et une surface annulaire interne 24 (comparable aux surfaces annulaires internes 7 et 16) convergeant au bord de-l'orifice de sortie 22 et assemblées l'une à l'autre le long de leurs bords arrière 25 et 26 pour former ledit orifice de sortie 22.

Dans le bord arrière 25 de la surface externe 23 sont découpées des échancrures 27 adjacentes au bord de l'orifice de sortie 22 et s'étendant dans ladite surface externe 23, à l'opposé dudit orifice de sortie 22.

On voit qu'ainsi les échancrures 27 sont borgnes, car obturées par la surface interne 24 et que, du côté interne, ladite partie arrière 21R est lisse puisque constituée par la surface interne 24, non échancrée.

Par ailleurs, comme cela est illustré sur la figure 1 par les flèches f, le générateur de flux chaud 10 peut être ventilé par de l'air circulant dans ledit système de capot 12, ledit air de ventilation étant prélevé sur le flux froid et pouvant sortir dudit capot au voisinage du bord de sortie 13. Lorsque la partie arrière 12R est remplacée par la partie arrière 21R des figures 2 et 3, l'air de ventilation est donc amené à sortir à travers les échancrures 27. Dans le cas où celles-ci seraient insuffisantes pour assurer un libre débit audit air de ventilation, on peut, comme représenté sur les figures 4 et 5, utiliser une partie arrière 28R en tous points identiques à la partie arrière 21R, à l'exception du fait que les surfaces externe et interne 23, 24 ne sont plus solidaires l'une de l'autre par leurs bords arrière 25, 26, le long du bord de l'orifice de sortie 22. En effet, entre les bords arrière 25 et 26 des surfaces externe et interne 23, 24 est alors ménagée une fente périphérique 29 entourant ledit orifice de sortie 22.

On augmente ainsi la surface de passage pour l'air de ventilation du générateur 10.

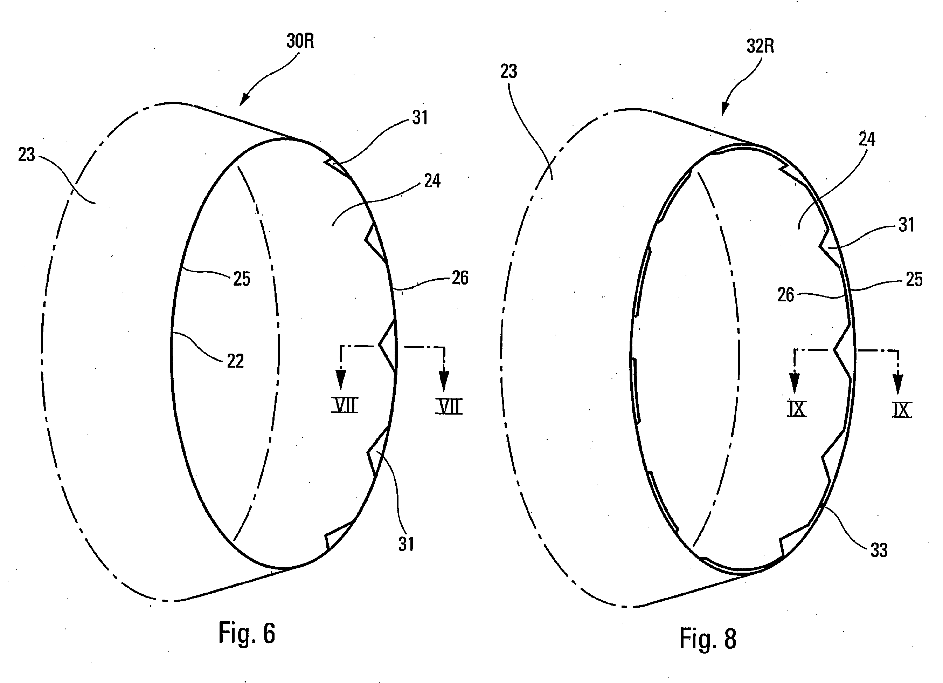

Sur les figures 6 et 7, on a représenté une partie arrière 30R semblable à la partie arrière 21R des figures 2 et 3, à l'exception du fait que les échancrures 27 dans le bord 25 de la surface externe 23 sont supprimées et remplacées par des échancrures 31 semblables découpées dans le bord 26 de la surface interne 24. De façon évidente, les échancrures 31 sont borgnes, car obturées par la surface externe 23.

De façon semblable, sur les figures 8 et 9, est représéntée une partie arrière 32R semblable à la partie arrière 28R des figures 4 et 5, de laquelle les échancrures 27 ont été supprimées et remplacées par les échancrures 31 de la partie arrière 30R des figures 6 et 7. Ainsi, entre les bords arrière 25 et 26 est formée une fente périphérique 33.

La figure 10 illustre un-mode de réalisation pratique 34R des parties arrière 21R et 30R des figures 2, 3 et 6, 7.

Dans la partie arrière 34R, on prévoit un arêtier 35 formant l'orifice de sortie 22, auquel sont rapportées des plaques ou tôles 36, 37 formant les surfaces 23, 24 en collaboration avec les faces planes 38 et 39 dudit arêtier 35. Dans l'une des faces 38, 39 dudit arêtier 35 sont découpés des évidements borgnes 40, représentatifs des échancrures 27, 31.

De la description ci-dessus, on comprendra aisément que les échancrures borgnes conformes à la présente invention peuvent résulter de perçages, d'évidements, d'enfoncements, d'estampage, de décolletage ou-de tout autre opération de formage ou d'usinage.

## Revendications

1. Turbomoteur à double flux comportant :
- une nacelle creuse (1) présentant un axe longitudinal (L-L) et comportant une partie avant d'entrée d'air (2) pourvue d'un bord d'attaque et une partie arrière de sortie d'air comprenant une surface annulaire externe et une surface annulaire interne convergeant l'une vers l'autre pour former un bord de fuite ;
- une soufflante (8) disposée dans ladite nacelle (1) en regard de ladite partie avant d'entrée d'air (2) et apte à engendrer le flux froid (9) dudit turbomoteur ;
- un générateur (10) disposé dans ladite nacelle (1), en aval de ladite soufflante (8), et apte à engendrer le flux chaud axial (11) dudit turbomoteur ; et
- un système de capot (12) entourant ledit générateur de flux chaud (10) et comportant une partie arrière comprenant une surface annulaire externe et une surface annulaire interne convergeant l'une vers l'autre pour former un orifice de sortie pour ledit flux chaud, ledit système de capot (12) délimitant avec la nacelle un canal à section annulaire (17) pour ledit flux froid (9) et ledit canal (17) se terminant par ladite partie arrière de sortie d'air de la nacelle dont le bord de fuite forme l'orifice de sortie dudit flux froid (9),
et comprenant des échancrures (27, 31, 40) aptes à atténuer le bruit de jet dudit turbomoteur à la périphérie de l'orifice de sortie (22) d'au moins l'un desdits flux (9, 11),
**caractérisé en ce que** lesdites échancrures (27, 31, 40) sont borgnes, du fait qu'elles entament l'une desdites surfaces annulaires convergentes (23, 24) formant ledit orifice de sortie (22) sans entamer l'autre desdites surfaces annulaires convergentes (23, 24).

2. Turbomoteur selon la revendication 1,
**caractérisé en ce que** lesdites échancrures borgnes (27, 40) entament ladite surface annulaire externe (23) sans entamer ladite surface annulaire interne (24).

3. Turbomoteur selon la revendication 1,
**caractérisé en ce que** lesdites échancrures borgnes (31, 40) entament ladite surface annulaire interne (24) sans entamer ladite surface annulaire externe (23).

4. Turbomoteur selon l'une des revendications 1 à 3,
**caractérisé en ce que** l'orifice de sortie (22) du flux chaud (11) est pourvu desdites échancrures borgnes (27, 31, 40).

5. Turbomoteur selon l'une des revendications 1 à 3,
**caractérisé en ce que** l'orifice de sortie (22) du flux froid (9) est pourvu desdites échancrures borgnes (27, 31, 40).

6. Turbomoteur selon les revendications 4 et 5,
**caractérisé en ce que** l'orifice de sortie du flux chaud (11) et l'orifice de sortie du flux froid (9) sont pourvus desdites échancrures borgnes (27, 31, 40).

7. Turbomoteur selon la revendication 4,
**caractérisé en ce que** ledit système de capot (12) est apte à être parcouru par de l'air de ventilation destiné à réguler la température dudit générateur de flux chaud (10), et à ce que ledit air de ventilation s'échappe au moins en partie à travers lesdites échancrures-borgnes (27, 31).

8. Turbomoteur selon la revendication 1, **caractérisé en ce que** l'orifice de sortie (22) dudit flux est délimité par un arêtier (35) solidaire desdites surfaces annulaires externe et interne convergentes (23, 24),
et que lesdites échancrures borgnes (27, 31) sont formées par des évidements (40) pratiqués dans l'une des faces dudit arêtier.

## Claims

1. Turbofan engine comprising:
- a hollow nacelle (1) having a longitudinal axis (L-L) and including a front air intake section (2), provided with a leading edge, and a rear air outlet section comprising an outer annular surface and an inner annular surface which converge toward one another to form a trailing edge;
- a fan (8) arranged in said nacelle (1) opposite said front air intake section (2) and designed to generate the cold stream (9) of said turbofan engine;
- a generator (10) arranged in said nacelle (1), downstream of said fan (8), and designed to generate the axial hot stream (11) of said turbofan engine; and
- a cowl system (12) surrounding said hot stream generator (10) and including a rear section which comprises an outer annular surface and an inner annular surface which converge toward one another to form an outlet orifice for said hot stream, said cowl system (12) defining with the nacelle a duct (17) of annular cross section for said cold stream (9), and said duct (17) being terminated by said rear air outlet section of the nacelle whose trailing edge forms the outlet orifice for said cold stream (9),
and comprising notches (27, 31, 40) designed to attenuate the jet noise of said turbofan engine at the periphery of the outlet orifice (22) for at least one of said streams (9, 11), **characterized in that** said notches (27, 31, 40) are blind, given that they cut into one of said convergent annular surfaces (23, 24) forming said outlet orifice (22) without cutting into the other of said convergent annular surfaces (23, 24).

2. The turbofan engine as claimed in claim 1, **characterized in that** said blind notches (27, 40) cut into said outer annular surface (23) without cutting into said inner annular surface (24).

3. The turbofan engine as claimed in claim 1, **characterized in that** said blind notches (31, 40) cut into said inner annular surface (24) without cutting into said outer annular surface (23).

4. The turbofan engine as claimed in one of claims 1 to 3, **characterized in that** the outlet orifice (22) for the hot stream (11) is provided with said blind notches (27, 31, 40).

5. The turbofan engine as claimed in one of claims 1 to 3, **characterized in that** the outlet orifice (22) for the cold stream (9) is provided with said blind notches (27, 31, 40).

6. The turbofan engine as claimed in claims 4 and 5, **characterized in that** the outlet orifice for the hot stream (11) and the outlet orifice for the cold stream (9) are provided with said blind notches (27, 31, 40).

7. The turbofan engine as claimed in claim 4, **characterized in that** said cowl system (12) is able to be flowing through by ventilating air that is intended to regulate the temperature of said hot stream generator (10), and **in that** said ventilating air escapes at least in part through said blind notches (27, 31).

8. The turbofan engine of claim 1, **characterized in that** the outlet orifice (22) for said stream is defined by a capping piece (35) secured to said convergent outer and inner annular surfaces (23, 24), and **in that** said blind notches (27, 31) are formed by cutouts (40) made in one of the faces of said capping piece.

## Patentansprüche

1. Mantelstromtriebwerk, Folgendes umfassend:
- eine hohle Gondel (1), die eine Längsachse (L-L) aufweist und die einen vorderen, mit einer Anströmkante ausgestatteten Lufteinlass-Abschnitt (2) umfasst und einen hinteren Luftauslass-Abschnitt, der eine Außenringfläche und eine Innenringfläche aufweist, die zur Bildung einer Hinterkante zueinander konvergieren,
- ein Gebläse (8), das in der Gondel (1) dem vorderen Lufteinlass-Abschnitt (2) gegenüberliegend angeordnet ist und das geeignet ist, den Kaltstrom (9) für das Triebwerk zu erzeugen,
- einen Generator (10), der in der Gondel (1) stromab des Gebläses (8) angeordnet ist und der geeignet ist, die heiße Axialströmung (11) des Triebwerks zu erzeugen und
- ein Haubensystem (12), das den Heißstrom-Generator (10) umgibt und einen hinteren Abschnitt umfasst, der eine Außenringfläche und eine Innenringfläche aufweist, die zueinander konvergieren, um eine Auslassöffnung für den Heißstrom zu bilden, wobei das Haubensystem (12) mit der Gondel einen Kanal mit ringförmigem Querschnitt (17) für den Kaltstrom (9) abgrenzt und der Kanal (17) mit dem hinteren Luftauslass-Abschnitt der Gondel endet, dessen Hinterkante die Auslassöffnung für den Kaltstrom (9) bildet
und Auskerbungen (27, 31, 40) aufweisend, die geeignet sind, den Strahllärm des Triebwerks am Umfang der Auslassöffnung (22) für mindestens einen der Ströme (9,11) zu unterdrücken,
**dadurch gekennzeichnet, dass** die Auskerbungen (27, 31, 40) blind sind, da sie eine der aufeinander zulaufenden, die Auslassöffnung (22) bildenden Ringflächen (23, 24) anschneiden, ohne die andere der aufeinander zulaufenden Ringflächen (23, 24) anzuschneiden.

2. Triebwerk nach Anspruch 1,
**dadurch gekennzeichnet, dass** die blinden Auskerbungen (27, 40) die Außenringfläche (23) anschneiden, ohne die Innenringfläche (24) anzuschneiden.

3. Triebwerk nach Anspruch 1,
**dadurch gekennzeichnet, dass** die blinden Auskerbungen (31, 40) die Innenringfläche (24) anschneiden, ohne die Außenringfläche (23) anzuschneiden.

4. Triebwerk nach einem der Ansprüche 1 bis 3,
**dadurch gekennzeichnet, dass** die Auslassöffnung (22) für den Heißstrom (11) mit den blinden Auskerbungen (27, 31, 40) ausgestattet ist.

5. Triebwerk nach einem der Ansprüche 1 bis 3,
**dadurch gekennzeichnet, dass** die Auslassöffnung (22) für den Kaltstrom (9) mit den blinden Auskerbungen (27, 31, 40) ausgestattet ist.

6. Triebwerk nach den Ansprüchen 4 und 5,
**dadurch gekennzeichnet, dass** die Auslassöffnung für den Heißstrom (11) und die Auslassöffnung für den Kaltstrom (9) mit den blinden Auskerbungen (27, 31, 40) ausgestattet ist.

7. Triebwerk nach Anspruch 4,
**dadurch gekennzeichnet, dass** das Haubensystem (12) geeignet ist, von Belüftungsluft durchströmt zu werden, die zur Regelung der Temperatur des Heißstrom-Generators (10) vorgesehen ist und **dadurch**, dass die Belüftungsluft zumindest teilweise durch die blinden Auskerbungen (27, 31) austritt.

8. Triebwerk nach Anspruch 1,
**dadurch gekennzeichnet, dass** die Auslassöffnung (22) des Stroms durch eine fest mit der Außen- und der Innenringfläche (23, 24) verbundene Nasenleiste (35) begrenzt ist und **dadurch**, dass die blinden Auskerbungen (27, 31) durch in der einen Seite der Nasenleiste ausgeführte Aussparungen (40) gebildet sind.
